# EUROPÄISCHE PATENTSCHRIFT

(11) **EP 2 395 989 B1**
(45) Veröffentlichungstag und Bekanntmachung des Hinweises auf die Patenterteilung: **24.12.2014**
(21) Anmeldenummer: 10710992.8
(22) Anmeldetag: 05.02.2010
(51) Int. Cl.: A61K 31/155, A61K 31/205, A61K 31/295, A61K 31/315, A61K 33/26, A61K 33/30, A61K 45/06, A61P 17/02

(54) **BEHANDLUNGSLÖSUNG FÜR DIE BEHANDLUNG VON WUNDEN, INSBESONDERE FÜR DIE LIQUIDE WUNDBEHANDLUNG**
LIQUID COMPOSITION FOR THE TREATMENT OF WOUNDS, IN PARTICULAR FOR THE LIQUID TREATMENT OF WOUNDS
COMPOSITION LIQUIDE POUR LE TRAITEMENT DES BLESSURES, EN PARTICULIER POUR LE TRAITEMENT LIQUIDE DES BLESSURES

(30) Priorität: 13.02.2009 DE 102009008919
(43) Veröffentlichungstag der Anmeldung: 21.12.2011
(73) Patentinhaber: Nawa Heilmittel GmbH, 90482 Nürnberg (DE)
(72) Erfinder: RIESINGER, Thomas, 90411 Nürnberg (DE)
(74) Vertreter: Graf Glück Kritzenberger
(86) Internationale Anmeldenummer: PCT/DE2010/000129
(87) Internationale Veröffentlichungsnummer: WO 2010/091661

(56) Entgegenhaltungen:
- EP-A1- 0 363 696
- WO-A1-00/33829
- WO-A1-03/004013
- FABRY W ET AL: "Bacterial decontamination of surgical wounds treated with Lavasept<(>R)" INTERNATIONAL JOURNAL OF HYGIENE AND ENVIRONMENTAL HEALTH, URBAN U. FISCHER, JENA, DE LNKD- DOI:10.1016/J.IJHEH.2006.03.008, Bd. 209, Nr. 6, 15. November 2006 (2006-11-15), Seiten 567-573, XP025184035 ISSN: 1438-4639 [gefunden am 2006-11-15]

## Beschreibung

Die Erfindung bezieht sich auf eine Behandlungslösung zur liquiden Wundbehandlung von akuten und chronischen Wunden, bestehend aus einer wässrigen Lösung zumindest enthaltend Zink, Eisen und Säure, wobei je Liter Lösung der Anteil an Zink 15 - 45 mg, der Anteil an Eisen 10 - 30 mg und der Anteil an Säure so gewählt ist, dass der pH-Wert der Behandlungslösung 2,75 bis 3,5 beträgt. Die zu behandelnde Wunde wird dabei mit einem ausreichend großen Volumen an Behandlungsflüssigkeit in einem liquiden Zustand gehalten, und zwar mit dem Ergebnis einer beschleunigten Wundheilung mit optimierter Narbenbildung.

Eine Behandlungslösung für die liquide Wundbehandlung ist grundsätzlich bekannt (DE 101 39 398 A1). Die bekannte Behandlungslösung enthält in wässriger Lösung Zink in Form von ZnCl2, Eisen in Form von FeSO₄ sowie eine anorganische Säure in solchen Anteilen, dass in der Lösung je Liter Flüssigkeit 10 bis 100 mg Zink, und 6,5 bis 65 mg Eisen enthalten sind und der pH-Wert der Lösung auf einen Wert zwischen 2,5 bis 3,5 eingestellt ist.

Es hat sich gezeigt, dass die bekannte Behandlungslösung zwar bei der Behandlung von postoperativen Wunden zu sehr guten Ergebnissen führt, sich solche Ergebnisse aber nicht generell bei der Wundbehandlung feststellen lassen, insbesondere auch nicht bei der Behandlung von akuten und chronischen Wunden.

Aus der Druckschrift WO 03/004013 A1 ist ein Wundbehandlungsmittel bekannt, welches in wässriger Lösung Polyhexamethylenbiguanid (PHMB) und ein Tensid umfasst. Die Druckschrift offenbart, dass der Anteil an Tensid 0,01 bis 1,5 Gewichts% und der Anteil an PHMB 0,01 bis 1,0 Gewichts% beträgt.

Aus der Druckschrift WO 00/33829 A1 sowie der Veröffentlichung FABRY W ET AL: "Bacterial decontamination of surgical wounds treated with Lavasept®" INTERNATIONAL JOURNAL OF HYGIENE AND ENVIRONMENTAL HEALTH, URBAN U. FISCHER, JENA, Bd. 209, Nr. 6, 15. November 2006, Seiten 567-573, ist eine Behandlungslösung bekannt, die ebenfalls ein Tensid und einen antiseptischen Anteil enthält.

Aufgabe der Erfindung ist es, eine Behandlungslösung aufzuzeigen, die generell bei der Behandlung von akuten und chronischen Wunden zu optimalen Behandlungsergebnissen, d.h. einer schnellen Wundheilung mit optimaler Narbenbildung führt.

Zur Lösung dieser Aufgabe ist eine Behandlungslösung für die liquide Wundbehandlung entsprechend dem Patentanspruch 1 ausgebildet.

Die erfindungsgemäße Behandlungslösung eignet sich insbesondere zur Behandlung von Hautlazerationen, Schnitt- und Schürfwunden sowie Risswunden, Dekubitaluzera, Arterio-venöse Ulzera, diabetische Ulzera, Verbrennungen I und II Grades und für die Befeuchtung von Verbänden und Wundauflagen, wie Kompressen, Gazen, Schwämmen, Alginaten, u.ä. sowie beim Verbandwechsel zum Lösen verkrusteter Verbände oder anderer verkrusteter Wundauflage.

Die erfindungsgemäße Behandlungslösung enthält in wässriger Lösung zusätzlich zu Eisen und Zink wenigstens das Tensid Cocamidopropyl Betain, sowie auch einen antiseptisch wirkenden Bestandteil bevorzugt in Form von Polyhexanid oder PHMB (Polyaminopropyl Biguanide).

Die Komponenten der erfindungsgemäßen Behandlungslösung unterstützen sich in synergetischer Weise, sodass die Behandlungslösung generell bei akuten und chronischen Wunden anwendbar ist und zu den gewünschten Ergebnissen führt.

Durch die spezielle Zusammensetzung der erfindungsgemäßen Behandlungslösung kommt es bei der Behandlung der jeweiligen Wunde zu einer synergetischen Wirkung der Komponenten in der Weise, dass zunächst durch das enthaltene Tensid ein Ablösung von Eiweißbestandteilen, nekrosen Gewebebestandteilen usw. aus der Wunde erfolgt, sodass dann in einer zweiten Stufe der antiseptische Bestandteil voll für ein erstes Abtöten von in der Wunde vorhandenen Keimen wirksam werden kann, so dass dann die Voraussetzungen für die Förderung der Wundheilung durch die Metallionen (Eisen und Zink) geschaffen sind und während der liquiden Behandlung die jeweilige Wunde durch die Metallionen auch keimfrei gehalten werden kann.

Generell enthält die erfindungsgemäße Behandlungslösung je Liter Lösungsmittel (insbesondere Wasser) die Bestandteile in folgenden Anteilen:

| | |
|---|---|
| Tensid: | 1000 bis 6000 mg |
| antiseptischer Bestandteil, insbesondere Polyhexanid: | 100 bis 1000 mg |
| Zink: | 15 bis 45 mg |
| Eisen: | 10 bis 30 mg. |

Bei einer bevorzugten Ausführung enthält die Behandlungslösung je Liter Lösungsmittel (insbesondere Wasser) die Bestandteile in folgenden Anteilen:

| | |
|---|---|
| Tensid: | etwa 3000 mg |
| antiseptischer Bestandteil, beispielsweise Polyhexanid: | etwa 200 mg |
| Zink: | 15 bis 45 mg |
| Eisen: | 10 bis 30 mg. |

Durch Zugabe einer Säure, vorzugsweise einer organischen Säure, z.B. durch Zugabe von Salzsäure ist der pH-Wert der Behandlungslösung jeweils auf einem Wert zwischen 2,75 und 3,5 eingestellt.

## Patentansprüche

1. Behandlungslösung zur liquiden Wundbehandlung von akuten und chronischen Wunden, bestehend aus einer wässrigen Lösung zumindest enthaltend Zink, Eisen und Säure, wobei je Liter Lösung der Anteil an Zink 15 - 45 mg, der Anteil an Eisen 10 - 30 mg und der Anteil an Säure so gewählt ist, dass der pH-Wert der Behandlungslösung 2,75 bis 3,5 beträgt, **dadurch gekennzeichnet, dass** die Lösung zusätzlich das Tensid Cocamidopropyl Betain sowie einen antiseptischen Bestandteil enthält, wobei der Anteil an Cocamidopropyl Betain 1000 bis 6000 mg und der Anteil des antiseptischen Bestandteils 100 bis 1000 mg betragen.

2. Behandlungslösung nach Anspruch 1, **dadurch gekennzeichnet, dass** der antiseptische Anteil Polyhexanid oder PHMB (Polyaminopropyl Biguanide) ist.

3. Behandlungslösung nach einem der vorhergehenden Ansprüche, **dadurch gekennzeichnet, dass** der Anteil an Tensid je Liter Lösung 3000 mg beträgt.

4. Behandlungslösung nach Anspruch 2, **dadurch gekennzeichnet, dass** der Anteil an Polyhexanid oder PHMB 200 mg beträgt.

5. Behandlungslösung nach einem der vorhergehenden Ansprüche, **dadurch gekennzeichnet, dass** sie je Liter Lösung die Komponenten in folgenden Anteilen enthält:
Zink: 15 - 45 mg
Eisen: 10 - 30 mg
Tensid: 3000 mg
antiseptischer Anteil: 200 mg.

## Claims

1. A treatment solution for the liquid wound treatment of acute and chronic wounds, consisting of an aqueous solution containing at least zinc, iron and acid, wherein the proportion of zinc is 15 - 45 mg per litre, the proportion of iron is 10 - 30 mg per litre and the proportion of acid is selected such that the pH of the treatment solution is 2.75 to 3.5, **characterized in that** the solution additionally contains the surfactant cocamidopropyl betaine as well as an antiseptic component, wherein the proportion of cocamidopropyl betaine is 1000 to 6000 mg and the proportion of the antiseptic component is 100 to 1000 mg.

2. The treatment solution according to claim 1, **characterized in that** the proportion of antiseptic is polyhexanide or PHMB (polyaminopropyl biguanide).

3. The treatment solution according to one of the preceding claims, **characterized in that** the proportion of surfactant is 3000 mg per litre of solution.

4. The treatment solution according to claim 2, **characterized in that** the proportion of polyhexanide or PHMB is 200 mg.

5. The treatment solution according to one of the preceding claims, **characterized in that** the solution contains the components in the following proportions per litre:
zinc: 15 - 45 mg
iron: 10 - 30 mg
surfactant: 3000 mg
proportion of antiseptic: 200 mg.

## Revendications

1. Solution traitante pour le traitement liquide de plaies, de plaies aigues et chroniques, constituée d'une solution aqueuse contenant au moins du zinc, du fer et de l'acide, par litre de solution, la part en zinc étant de 15 à 45 mg, la part en fer étant de 10 à 30 mg et la part en acide étant choisie de telle sorte que la valeur pH de la solution traitante soit de 2,75 à 3,5, **caractérisée en ce que** la solution contient en supplément l'agent tensioactif bétaïne cocamidopropyle, ainsi qu'un constituant antiseptique, la part en bétaïne de cocamidopropyle étant de 1000 à 6000 mg et la part en constituant antiseptique étant de 100 à 1000 mg.

2. Solution traitante selon la revendication 1, **caractérisée en ce que** la part antiseptique est de la polyhexanide ou du PHMB (polyaminopropyl biguanide).

3. Solution traitante selon l'une quelconque des revendications précédentes, **caractérisée en ce que** la part en agent tensioactif est de 3000 mg par litre de solution.

4. Solution traitante selon la revendication 2, **caractérisée en ce que** la part en polyhexanide ou en PHMB est de 200 mg.

5. Solution traitante selon l'une quelconque des revendications précédentes, **caractérisée en ce que** par litre de solution, elle contient les composants dans les parts suivantes :
zinc : de 15 à 45 mg
fer : de 10 à 30 mg
agent tensioactif : 3000 mg
part d'antiseptique : 200 mg.
